# EUROPÄISCHE PATENTANMELDUNG

(11) **EP 2 514 443 A1**
(43) Veröffentlichungstag der Anmeldung: **24.10.2012**
(21) Anmeldenummer: 12002735.4
(22) Anmeldetag: 19.04.2012
(51) Int. Cl.: A61L 2/07, A23L 3/015, A23L 3/16

(54) **Entkeimungsverfahren**

(30) Priorität: 21.04.2011 DE 102011018652
(71) Anmelder: Hochschule Ostwestfalen-Lippe, 32657 Lemgo (DE)
(72) Erfinder: Müller, Ulrich, Prof. Dr.-Ing., 32657 Lemgo (DE); Wilhelm , Patrick, Dipl.-Ing., 32257 Bünde (DE)
(74) Vertreter: Holland, Ralf

(57) **Zusammenfassung**

Bei einem Entkeimungsverfahren für insbesondere getrocknete pflanzliche Materialpartikel, bei dem die Materialpartikel in einem druckfesten Behälter 1 einer Dampfphase ausgesetzt werden, wird bei einer Evakuierung des Behälters 1 die Dampfphase gegen ein trockenes Spülgas ausgetauscht.

## Beschreibung

Die Erfindung betrifft ein Entkeimungsverfahren für insbesondere getrocknete pflanzliche Materialpartikel, bei dem die Materialpartikel in einem druckfesten Behälter einer Dampfphase ausgesetzt werden.

Die Reduzierung von an Materialien anhaftenden Mikroorganismen wie Keimen und Sporen ist oft zweckmäßig, häufig sogar aufgrund gesetzlicher Vorschriften notwendig. Abhängig von dem zu entkeimenden Material wurden vielfältige thermische Verfahren, mechanische Verfahren, chemische Verfahren und Kombinationen derselben entwickelt.

Bei den thermischen Verfahren der Entkeimung von pflanzlichen Materialien, beispielsweise Gewürzen, Arzneidrogen oder dergleichen pulver- bis granulatartig, zumeist getrocknet vorliegender Rohware, hat sich die Sattdampfmethode durchaus bewährt, mit der vorhandene Keime, zumeist bakterielle Sporen, durch die entstehende, feuchte Hitze abgetötet werden. Hierzu werden in der Regel Sattdampftemperaturen von über 120°C und lange Einwirkzeiten von mehreren Minuten benötigt. Da bakterielle Sporen, die einen Großteil der oberflächlich anhaftenden Keime ausbilden, durch einen Wasserzutritt aktiviert und damit empfindlich werden, sind Sattdampfentkeimungsverfahren aufgrund der Kondensation des Wassers auf dem Material und damit einer schnellen Wärmeübertragung durchaus effektiv und in vielfältigen Formen etabliert. Diese hitze-thermischen, abtötenden Verfahren werden als klassisch bezeichnet.

Als nachteilig bei den bekannten Sattdampfverfahren ist jedoch gerade die thermische Einwirkung auf das zu behandelnde pflanzliche Material anzusehen, dem bei der Entkeimung wertgebende Inhaltsstoffe regelmäßig abgehen. Dies ist insbesondere dann der Fall, wenn die pflanzlichen Materialien Ölzellen aufweisen, da es durch die Sattdampfbehandlung zu einer Zerstörung der Ölzellenmembran kommen kann. Dies gilt insbesondere bei obenflächennahen Ölzellen, wie sie beispielsweise bei Majoran vorhanden sind. So kann es bei der Entkeimung von Majoran mit bekannten Entkeimungsverfahren zu einem Verlust der ätherischen Öle von bis zu 90% kommen.

Ein weiterer, gravierender Nachteil aller nassen Entkeimungsverfahren und damit auch der Sattdampfentkeimung ist das Problem der anschließenden Trocknung der behandelten Materialien, für die es zumeist noch einer Umsetzung des Materials aus einem Behälter für die Dampfbeaufschlagung wie einem Autoklaven oder dergleichen in eine Trocknungsvorrichtung bedarf. Darüber hinaus wird auch eine noch so schonende Trocknung den pflanzlichen Materialien weitere, wertvolle Inhaltsstoffe entziehen, einhergehend mit Farb- und Geschmacksveränderungen.

Bei einem in der Literatur als Lemgoer Sattdampfverfahren bekannt gewordenem Verfahren, weiter dokumentiert in der Dissertation von Markus Lilie, Universität Bielefeld 2009, erfolgt nach einer vergleichsweise milden, kurzzeitigen Bedampfung bei niedrigen Temperaturen eine sehr schnelle Evakuierung, so dass Keime aufgrund der spontanen Verdampfung, einer sogenannten Flash-Verdampfung, mechanisch von den Oberflächen der Materialpartikel gerissen werden. Dieses Verfahren weist eine hohe Entkeimungseffizienz auf, da durch die zunächst stattfindende Kondensation die Haftkräfte zwischen den Keimen und den Materialoberflächen vermindert und danach die Keime durch die schnelle Evakuierung mitgerissen werden. Jedoch werden auch feine pflanzliche Partikel durch die sehr hohen Strömungsgeschwindigkeiten bei dem sehr schnellen Evakuierungsvorgang mitgerissen. Aufgrund der kurzen Bedampfung sind ferner die hitze-thermischen Abtötungseffekte gering. Darüber hinaus bleibt auch bei diesem Entkeimungsverfahren das Problem bei Ölzellen aufweisenden pflanzlichen Partikel bestehen, denn auch bei diesem Verfahren werden die Ölzellenmembrane in hohem Maße zerstört werden, einhergehend mit einem entsprechenden Verlust ätherischer Öle oder anderer, in vergleichbaren Zellen eingebundener, wertvoller Substanzen.

Vor diesem technischen Hintergrund macht die Erfindung es sich zur Aufgabe, ein äußerst materialschonendes und dennoch sehr wirkungsvolles Entkeimungsverfahren zur Verfügung zu stellen.

Gelöst wird diese technische Problematik durch ein Entkeimungsverfahren für insbesondere getrocknete pflanzliche Materialpartikel, bei dem die Materialpartikel in einem druckfesten Behälter, vorzugsweise nach einer Vorabevakuierung, einer Dampfphase ausgesetzt werden, gemäß des Anspruchs 1 durch die Maßnahme, dass bei einer Evakuierung des Behälters die Dampfphase gegen ein trockenes Spülgas ausgetauscht wird.

Durch den Austausch der Dampfphase gegen ein trockenes Spülgas wird gleichsam ein Gegendruck aufgebaut, so dass es durch die Evakuierung zu keiner Ölzellen zerstörenden Ausdehnung des Öls kommt, durch die die Membranen der Ölzellen überdehnt werden, platzen und das wertvolle ätherische Öl entweicht.

Neben der keimtötenden Wirkung durch die thermische Einwirkung während der Bedampfungsphase kommt es jedoch auch hier zu einer mechanischen Ablösung von Sporen bzw. deren abgetöteten Reste, wenn durch geeignete Wahl der Prozessparameter erreicht wird, dass es lokal auf den Oberflächen der Materialpartikel zu einer spontanen Verdampfung kommt.

Durch ein schnelles Fluten des Gefäßes mit dem trockenen Spülgas entsteht in kurzer Zeit ein großes Partialdruckgefälle des Wassers auf der Oberfläche eines Materialpartikels und der Umgebung und ruft damit lokal und zeitlich begrenzt eine Vielzahl von die Keime ablösenden, spontanen Flash-Verdampfungen hervor.

Durch diese lokale und zeitlich begrenzte spontane Verdampfung erfährt das Material insgesamt eine schonende Behandlung während einer Entkeimung, die trotzdem so effizient ist, dass die Einwirkzeit der Dampfphase derart gering bemessen werden kann, dass die von den bedampften Materialpartikeln angenommene Temperatur geringer oder gleich der Dampftemperatur ist.

So kann beispielsweise erreicht werden, dass bei einer Dampftemperatur von 125° und einer Manteltemperatur des Gefäßes von 20°C die Temperatur eines zu behandelnden pflanzlichen Materials nur auf 80°C steigt. Dennoch kann der Keimgehalt eines mit dem erfindungsgemäßen Verfahren behandelten pflanzlichen Materials regelmäßig unter die Nachweisgrenze gesenkt werden.

Entsprechend kurz kann die Einwirkzeit der Dampfphase bemessen werden, in der Regel weniger als eine Minute, zumeist nur wenige 10 Sekunden.

Als zweckmäßig bei dem Verfahren nach der Erfindung hat es sich erwiesen, wenn die Materialpartikel verwirbelt werden. Eine Verwirbelung wird zwar in geringem Umfang bereits mit dem Einbringen des Dampfes in das vorab evakuierte Gefäß erreicht, jedoch kann mit dem Eintrag des Spülgases insbesondere von unten in den Behälter erreicht werden, dass sich eine Wirbelschicht ausbildet. Neben derartigen pneumatischen Verwirbelungen kann alternativ auch an eine mechanische Verwirbelung gedacht werden, beispielsweise durch rotierende Mischerarme.

Bevorzugt ist vorgesehen, die Prozessparameter so einzustellen, dass bei dem Austausch der Dampfphase gegen das Spülgas der Druck in dem Behälter weitgehend konstant bleibt oder ansteigt, bspw. bis auf einen dann konstant gehaltenen Wert, abhängig von dem von dem Material benötigten Gegendruck. Infolge dieser Maßnahmen werden auch sehr feine Partikel des Materials bei der Evakuierung nicht aus dem Behälter ausgetragen, sondern verbleiben in diesem. Daneben wird durch diese Maßnahme in hohem Maße sichergestellt, dass es nur lokal und zeitlich begrenzt zu der spontanen Verdampfung, der Flash-Verdampfung, kommt. Insbesondere wird auch bei unterschiedlichem Material eine schonende Entkeimung sichergestellt, bei der es bei geeigneter Wahl des Gegendrucks eben zu keiner Beschädigung der Membranen von Ölzellen kommt.

Andererseits kann durch ein vergleichsweise schnelles Ausströmen des Spülgases und des Dampfes bei der Evakuierung erreich werden, dass mit der Evakuierung des Behälters ein Materialaustrag erfolgt. Das Material kann dann an einer geeigneten Scheidevorrichtung abgefangen werden.

Ist das Spülgas ausreichend warm, kann durch dessen Eintrag in den Behälter, gegebenenfalls auch über Zeitdauer des Austausches der Dampfphase hinweg, eine Trocknung der Materialpartikel erfolgen. Da hierdurch ein Umsetzen des Materials in eine Trockenvorrichtung entfällt, wird das Material durch diese Maßnahme weiter geschont.

Dabei kann das Spülgas in einfacher Weise eine Druckluft sein, wie sie in fast allen Entkeimungsverfahren nutzenden Betrieben vorhanden ist.

Das Wesen der Erfindung wird anhand der Zeichnung und anhand von Ausführungsbeispielen weiter erläutert. In der Zeichnung zeigt:
- Fig. 1:: einen Vergleich der Entwicklung der aeroben mesophilen Keimzahl bei dem Lemgoer Verfahren und dem erfindungsgemäßen Verfahren bei konstanter Dampftemperatur über der Zeit,
- Fig. 2:: den Vergleich der aeroben mesophilen Sporenzahl aus dem selben Versuch,
- Fig. 3:: die Entwicklung der aeroben mesophilen Keimzahl über der Bedampfungszeit bei dem erfindungsgemäßen Verfahren bei unterschiedlichen Dampftemperaturen,
- Fig. 4:: die Entwicklung der aeroben mesophilen Sporenzahl über der Bedampfungszeit bei dem erfindungsgemäßen Verfahren bei unterschiedlichen Temperaturen und
- Fig. 5:: schematisch eine Vorrichtung zur Durchführung des erfindungsgemäßen Verfahrens.

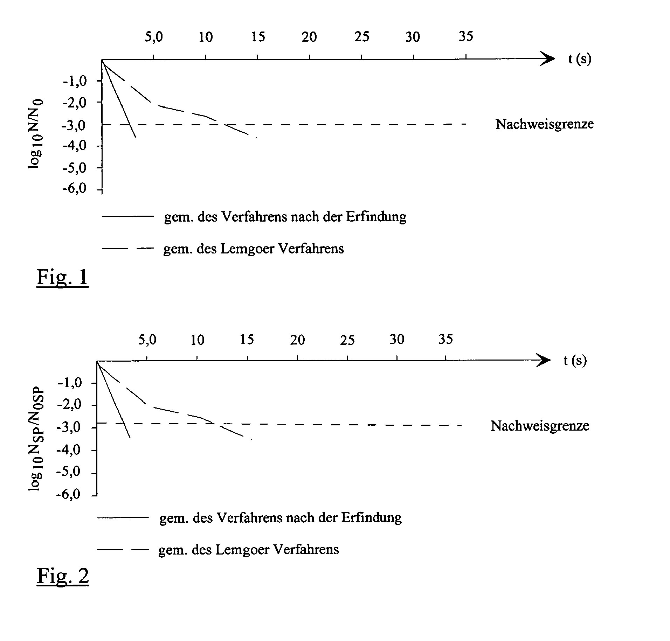

Die Figuren 1 und 2 zeigen einen Vergleich des sogenannten Lemgoer Verfahrens, gestrichelt dargestellt, mit dem erfindungsgemäßen Verfahren mit einer Trockenluftspülung, durchgezogene Linie, bei der Entkeimung von Majoran.

Die Sattdampftemperatur bei der Verfahrensdurchführung betrug in allen Fällen 115°C und die Einwirkzeit bei dem Lemgoer Verfahren 5 Sekunden, 10 Sekunden und 25 Sekunden sowie bei dem erfindungsgemäßen Verfahren 5 Sekunden, 10 Sekunden, 30 Sekunden und 50 Sekunden. Nach jeder Einwirkzeit wurde jeweils eine Dreifachbestimmung der aeroben mesophilen Keimzahl N, der sogenannten Gesamtkeimzahl, und der aeroben mesophilen Sporenzahl N_{SP} bestimmt. Die Sporen sind sehr hitzeresistente Überdauerungsformen der Bakteriengattungen Bacillus und Clostridium, wobei letztere anaerob sind und folglich nicht bei den Aerob, mesophilen Sporen mitgezählt werden.

Aus der Anfangskeimzahl N₀ bzw. der Anfangssporenzahl N_{0SP} und der Keimzahl N bzw. der Sporenzahl N_{SP} lässt sich die Überlebensrate log₁₀ N/N₀ bzw. log₁₀ N_{SP}/N_{0SP} für jede Sattdampftemperatur und Bedampfungszeit ermitteln. Diese versuchsergebnisse sind in den Figuren 1 und 2 jeweils gegenüber gestellt.

Bereits bei dem Lemgoer Verfahren konnte gegenüber den klassisch-abtötenden eine erhebliche Steigerung der Keimreduktion verzeichnet werden, bspw. wurden bei 10 s Bedampfungszeit mit einem Dampf von 125°C von mit B. subtilis beimpften Modellmaterialien mit anschließender schneller Evakuierung vier Dekaden, log-Stufen, einer Keimzahlreduzierung erreicht, während bei hitzethermisch abtötenden Verfahren höchstens eine Dekade einer Keimzahlreduzierung zu verzeichnen war.

Das erfindungsgemäße Verfahren mit dem Austausch der Dampfphase durch ein Spülgas zeigt darüber hinaus im Vergleich mit dem Lemgoer Verfahren bei einer Bedampfungstemperatur von 115°C und einer Bedampfungszeit von 5 Sekunden um ca. drei Dekaden bessere Entkeimungsergebnisse, sowohl bei der aeroben mesophilen Keimzahl gem. Fig. 1 als auch bei der aeroben mesophilen Sporenzahl gem. Fig. 2.

Die nur leicht unterschiedlichen Ergebnisse in den Figuren 1 und 2 werden darauf zurückgeführt, dass bei der Bestimmung der Überlebensrate der aeroben mesophilen Keimzahl Nichtsporen bei der Temperatur von 115°C in kürzester Zeit abgetötet wurden und dass überwiegend die hitzeresistenten Sporen der Bakteriengattungen Bacillus und Clostridium auf dem Majoran vorhanden waren, wie es bei trocknen pflanzlichen Materialien vielfach der Fall ist.

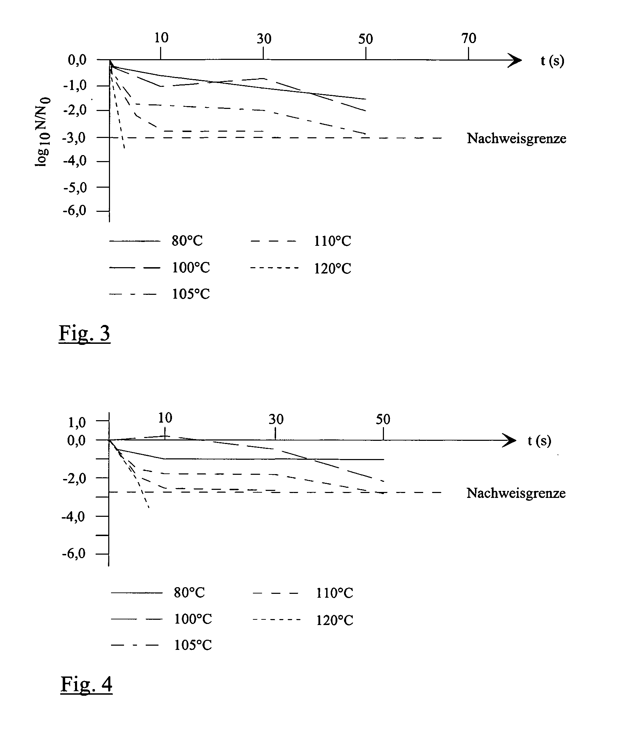

Dies bestätigen im Wesentlichen auch die in den Figuren 3 und 4 wiedergegebenen Messergebnisse von fünf Einzelentkeimungen von Majoranproben bei Sattdampf von 80°C, 100°C, 110°C und 120°C. Von jeder Probe wurde per Doppelbestimmung die aerobe mesophile Gesamtkeimzahl N ermittelt. Aus N₀ und N wurde die Überlebensrate log₁₀ N/N₀ pro Gramm Majoran für jede Sattdampftemperatur und Bedampfungszeit bestimmt und sind die entsprechenden Ergebnisse in Figur 3 für die aerobe mesophile Keimzahl und entsprechend die Überlebensrate log₁₀ N_{SP}/N_{0SP} pro Gramm Majoran für die aerobe mesophile Sporenzahl in Figur 4 wiedergegeben.

Die Figuren 3 und 4 zeigen, dass bei einer Dampftemperatur von 110°C bei dem erfindungsgemäßen Verfahren schon nach weniger als 5 Sekunden die Nachweisgrenze und damit eine Reduzierung der Keime um drei Dekaden im Wesentlichen erreicht wurde.

Aufgrund dieser kurzen Einwirkzeit der Dampfphase haben die Materialpartikel des Majorans die Sattdampftemperatur noch nicht erreicht, womit die thermische Belastung des Materials gegenüber den thermischen Abtötungsverfahren über einen erheblich längeren Zeitbedarf hinweg sehr gering ist.

Die hohe Überlebensrate der aeroben mesophilen Sporenzahl gemäß Figur 4 bei 80°C und 100°C wird auf das Fehlen der vegetativen Keime gegenüber der Überlebensrate der aeroben mesophilen Keimzahl zurückgeführt.

Den Figuren 3 und 4 lässt sich ferner eine gewisse Zweistufigkeit des Verfahrens nach der Erfindung entnehmen, eine schnelle und starke Reduktion insbesondere der Sporenzahl innerhalb den ersten 10 Sekunden auf ein danach nahezu konstantes Niveau.

Die Ausnahme bei einer Sattdampftemperatur von 110°C, bei der nach 30 Sekunden eine weitere Reduktion festzustellen war, dürfte auf thermisch abtötende Effekte zurückgeführt werden können.

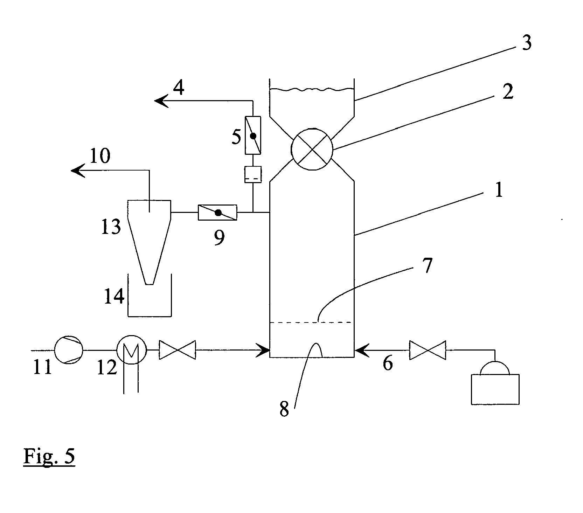

Figur 5 zeigt unter Verwendung der einschlägigen Symbole eine Vorrichtung für die Durchführung des erfindungsgemäßen Entkeimungsverfahrens in einem druckfesten Behälter 1, der über eine Zellenradschleuse 2, gegebenenfalls doppelt ausgebildet, mit nur angedeuteten, zu entkeimenden Materialpartikeln aus einem Vorratsbehälter 3 beschickt wird.

Nach Schließen der Zellenradschleuse 2 kann der druckfeste, säulenartige Behälter 1 gemäß Pfeil 4 evakuiert werden. Nach Schließen einer Klappe 5, eines Ventils oder dergleichen erfolgt die Beaufschlagung des in den Behälter 1 eingebrachten Materials mit Sattdampf von unten gemäß Pfeil 6. Bereits bei diesem Verfahrensschritt erfolgt eine gewisse Verwirbelung des eingebrachten Materials durch den einschießenden Dampf, gegebenenfalls erfolgt der Dampfeintrag aber auch in bereits bspw. mechanisch durch Mischerarme verwirbelte Materialpartikel.

Solches wird durch die Maßnahme erleichtert, dass eine gitterartige Struktur 7 das eingebrachte Material davon abhält, auf den Boden 8 des Behälters 1 abzusinken.

Nach Öffnen bspw. einer Klappe 5 erfolgt die Evakuierung des Dampfes gemäß Pfeil 4 aus dem Behälter 1, die im Vergleich mit Evakuierungen bei dem Lemgoer Verfahren sehr moderat erfolgt. Im Zuge der Evakuierung wird ein die Dampfphase verdrängendes Spülgas, beispielsweise Druckluft, bodenseitig, unterhalb der gitterartigen Struktur 7 bei fehlender Druckluft mittels eines Kompressors 11, gegebenenfalls über einen Wärmetauscher 12 vorgewärmt, dem Behälter 1 derart zugeführt, dass der Gesamtdruck innerhalb des Behälters 1 im wesentlichen zumindest gleich bleibt, gegebenenfalls materialabhängig auch höher eingestellt wird. Die mechanische Entkeimung der Materialpartikeln wird weiterhin aufgrund der großen Partialdruckdifferenzen lokal und zeitlich begrenzt erfolgen.

Alternativ kann eine Evakuierung und insbesondere eine Entleerung des Behälters 1 auch gemäß Pfeil 10 nach Öffnen einer Klappe 9 über eine Scheidevorrichtung 13 erfolgen, beispielsweise ein Zyklon, in der die von dem keimreichen Dampf, vermischt mit dem Spülgas, bei der Evakuierung mitgerissene Materialpartikel separiert und in einem Behälter 14 gesammelt werden. Es kann so ein Materialaustrag aus dem Behälter 1 auch erreicht werden.

Alternativ, bei geringen Strömungsgeschwindigkeiten während der Evakuierung, gegebenenfalls unter Verzicht auf die Scheidevorrichtung 13, kann erreicht werden, dass auch feinpulvriges, in den Behälter 1 eingebrachtes Material in diesem verbleibt.

Mit dem Einbringen des Spülgases, gegebenenfalls über die Zeit der eigentlichen Evakuierung hinaus, kann ferner in den Behälter 1 eingebrachtes Material noch getrocknet werden.

## Patentansprüche

1. Entkeimungsverfahren für insbesondere getrocknete pflanzliche Materialpartikel, bei dem die Materialpartikel in einem druckfesten Behälter einer Dampfphase ausgesetzt werden, **dadurch gekennzeichnet, dass** bei einer Evakuierung des Behälters die Dampfphase gegen ein trockenes Spülgas ausgetauscht wird.

2. Entkeimungsverfahren nach Anspruch 1, **dadurch gekennzeichnet, dass** der Behälter vor der Beaufschlagung mit einer Dampfphase evakuiert wird.

3. Entkeimungsverfahren nach Anspruch 1 oder 2, **dadurch gekennzeichnet, dass** es lokal auf der Oberfläche der Materialpartikel zu einer spontanen Verdampfung kommt.

4. Entkeimungsverfahren nach einem oder mehreren der vorangehenden Ansprüche, **dadurch gekennzeichnet, dass** die Einwirkzeit der Dampfphase derart gering bemessen ist, dass die von den bedampften Materialpartikeln angenommene Temperatur geringer oder gleich der Dampftemperatur ist.

5. Entkeimungsverfahren nach einem oder mehreren der vorangehenden Ansprüche, **dadurch gekennzeichnet, dass** die Einwirkzeit weniger als eine Minute beträgt.

6. Entkeimungsverfahren nach einem oder mehreren der vorangehenden Ansprüche, **dadurch gekennzeichnet, dass** die Materialpartikel verwirbelt werden.

7. Entkeimungsverfahren nach einem oder mehreren der vorangehenden Ansprüche, **dadurch gekennzeichnet, dass** bei dem Austausch der Dampfphase gegen das Spülgas der Druck in dem Behälter konstant bleibt oder ansteigt.

8. Entkeimungsverfahren nach einem oder mehreren der vorangehenden Ansprüche, **dadurch gekennzeichnet, dass** mit dem Austrag der Dampfphase aus dem Behälter ein Materialaustrag erfolgt.

9. Entkeimungsverfahren nach einem oder mehreren der vorangehenden Ansprüche, **dadurch gekennzeichnet, dass** durch den Eintrag des Spülgases eine Trocknung der Materialpartikel erfolgt.

10. Entkeimungsverfahren nach einem oder mehreren der
vorangehenden Ansprüche, **dadurch gekennzeichnet, dass** das Spülgas Druckluft ist.
